(19) Europäisches Patentamt
European Patent Office
Office européen des brevets

(11) Numéro de publication: **0 125 986**
**B1**

# (12) FASCICULE DE BREVET EUROPEEN

(45) Date de publication du fascicule du brevet: **15.07.87**

(51) Int. Cl.[4]: **C 25 B 3/02,** C 07 H 19/00, C 07 H 7/02

(21) Numéro de dépôt: **84400925.8**

(22) Date de dépôt: **04.05.84**

## (54) Procédé de préparation des cétones correspondant aux 1,4-3,6-dianhydrohexitols par électrooxydation anodique.

(30) Priorité: **04.05.83 FR 8307458**

(43) Date de publication de la demande: **21.11.84 Bulletin 84/47**

(45) Mention de la délivrance du brevet: **15.07.87 Bulletin 87/29**

(84) Etats contractants désignés: **CH DE GB LI**

(56) Documents cités: **EP - A - 0 040 331**

(73) Titulaire: **Roquette Frères, F-62136 Lestrem (FR)**

(72) Inventeur: **Fleche, Guy, 49, rue Georges Charlet "Le Sart", F-59660 Merville (FR)**
Inventeur: **Gaset, Antoine, 75, allée de Brienne, F-31000 Toulouse (FR)**
Inventeur: ***Jacquet, Fabienne, 42, rue des Couteliers,* F-31000 Toulouse (FR)**

(74) Mandataire: **Koch, Gustave et al, Cabinet PLASSERAUD 84, rue d'Amsterdam, F-75009 Paris (FR)**

**EP 0 125 986 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

L'invention a pour objet un procédé de préparation des cétones correspondant aux 1,4-3,6-dianhydrohexitols.

Il est rappelé que les cétones en question dérivent desdits 1,4-3,6-dianhydrohexitols par oxydation des seules fonctions hydroxyle endo; or, le dianhydromannitol ou isomannide comporte deux fonctions hydroxyle endo et le dianhydrosorbitol ou isosorbide en comporte une.

L'isomannide et l'isosorbide sont obtenus à partir des hexitols correspondants en éliminant deux molécules d'eau par hexitol; cette élimination d'eau peut être réalisée en présence par exemple d'acides minéraux forts ou de résines échangeuses d'ions fortement acides.

L'isomannide et l'isosorbide ont la même formule plane suivante:

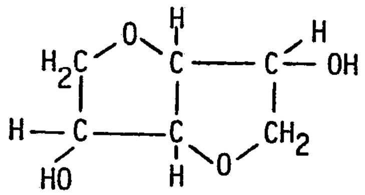

et se distinguent par le fait que:

– l'isomannide a ses deux hydroxyles en position endo,

– l'isosorbide ayant l'un de ses deux hydroxyles (celui qui est lié au carbone en position 5) en position endo, l'autre étant en position exo.

Du fait que l'hydroxyle endo peut former une liaison hydrogène avec l'oxygène du groupe éther de l'autre noyau, l'isomannide comporte deux liaisons hydrogène et l'isosorbide une seule.

L'oxydation de l'un seulement des deux groupes hydroxyle endo de l'isomannide conduit à une monocétone, le 1,4-3,6-dianhydro-D-fructose, celle des deux groupes hydroxyle endo à une dicétone, le 1,4-3,6-dianhydro-D-thréo-2,5-hexodiulose.

L'oxydation de l'unique groupe hydroxyle endo de l'isosorbide conduit à une monocétone, à savoir le 1,4-3,6-dianhydro-L-sorbose.

Ces trois composés constituent des produits précieux en tant qu'intermédiaires de synthèse.

Il est déjà connu d'oxyder les hydroxyles endo des dianhydrohexitols en fonction cétone par action de l'oxygène en présence d'un catalyseur à base de métal noble (notamment platine/charbon).

L'inconvénient de cette réaction d'oxydation catalytique réside dans le fait que:

– le rendement de conversion est moyen, ne dépassant pas environ 40% dans le cas de l'isosorbide,

– seules des solutions faiblement concentrées en dianhydrohexitol de départ peuvent être traitées.

L'invention a pour but, surtout, de remédier à ces inconvénients et de fournir un procédé de préparation des dérivés cétoniques de l'isomannide et de l'isosorbide qui réponde mieux que celui qui existe déjà aux diverses exigences de la pratique.

Or, la Société Demanderesse a trouvé que ce but pouvait être atteint en soumettant l'isomannide et l'isosorbide à une électrooxydation anodique.

Il s'ensuit que, conformément à l'invention, le procédé de préparation des dérivés cétoniques de l'isomannide et de l'isosorbide, est caractérisé par le fait que l'on soumet à une électrooxydation anodique une solution aqueuse d'isomannide ou d'isosorbide comportant un bromure alcalin à titre électrolyte, le pH de cette solution étant de 6 à 8.

Suivant un mode de réalisation avantageux, le procédé de préparation conforme à l'invention est caractérisé par le fait que l'électrooxydation anodique est mise en œuvre, en présence de NaBr à une concentration de 0,5 à 11% en poids de préférence de 1 à 5% en poids, sur une solution d'isomannide ou d'isosorbide d'une concentration de 0,5 à 40%, de préférence de 15 à 25% en poids, et la température de 10 à 60 °C, de préférence de 15 à 20 °C, la quantité d'électricité appliquée étant de 50 000 à 500 000, de préférence de 300 000 à 500 000 coulombs par mole de dianhydrohexitol traité.

Suivant un autre mode de réalisation avantageux, la réaction conforme à l'invention est mise en œuvre dans une cellule électrolytique comportant des électrodes de préférence en platine ou en carbone, les compartiments anodique et cathodique pouvant éventuellement être séparés par un diaphragme.

D'autres caractéristiques de l'invention apparaîtront dans la suite de la description qui se rapporte à des modes de réalisation avantageux.

Se proposant par conséquent d'oxyder en fonction cétone les groupements hydroxyle endo de l'isomannide et de l'isosorbide, on s'y prend comme suit ou de façon équivalente.

On prépare des solutions aqueuses d'isomannide et d'isosorbide d'une concentration de 0,5 à 40% en poids, de préférence de 15 à 25% en poids, en dissolvant le dianhydrohexitol en quantité convenable dans de l'eau.

On ajoute à la solution ainsi formée, un électrolyte qui est avantageusement constitué par un halogénure alcalin; de préférence, on a recours à un bromure, notamment du NaBr que l'on dissout en une quantité telle que la concentration de la sulution en bromure est amenée à 0,5 à 11%, de préférence de 1 à 5% en poids.

On ajuste le pH de la solution à une valeur de 5 à 10, de préférence de 6 à 8, plus préférentiellement à 7.

On introduit la solution ainsi préparée dans la cuve d'une cellule d'électrolyse comportant des électrodes, de préférence en platine ou en carbone, on amène sa température à une valeur de préférence proche de l'ambiante, notamment de 15 à 20 °C, et on applique entre les électrodes une différence de potentiel suffisante pour faire parcourir l'électrolyte contenant le dianhydrohexitol par un courant électrique, de façon à lui apporter

une quantité d'électricité suffisante, choisie de préférence, entre environ 400 000 et 500 000 coulombs/mole pour avoir un rendement optimal.

L'expérience a prouvé que la fonction hydroxyle «exo» de l'isosorbide n'était pas oxydée dans ces conditions (cette constatation a été confirmée dans le cadre d'une expérience effectuée sur le troisième dianhydrohexitol de même formule plane que les deux qui précèdent, à savoir le dianhydroiditol; en effet, le dianhydroiditol ne subit aucune oxydation de ses deux fonctions hydroxyle qui sont toutes deux en position exo).

Dans les essais destinés à permettre la détermination des meilleures conditions expérimentales et dont il va être question ci-après, on désigne par:

– rendement, dans le cas de l'isosorbide, le rapport

$$\frac{\text{nombre de moles de cétone formée}}{\text{nombre de moles de dianhydrohexitol de départ}} \times 100$$

(le fait que, dans le cas de l'isomannide, il y a formation d'une mono- et d'une dicétone, amène à considérer deux rendements)

Rendement faradique, le rapport

$$\frac{\text{quantité d'électricité theorique}}{\text{quantité d'électricité expérimentale}} \times 100$$

dans lequel:

a) la quantité d'électricité théorique ou Qi.th. est bien connue par la formule

$$Qi.th. = a \times F \times n$$

avec

F = 96 500 coulombs

n = nombre de moles de dérivé obtenu

a = nombre d'électrons mis en jeu

b) la quantité d'électricité expérimentale ou Qi.exp. est donnée par la formule

$$Qi.exp. = I \times t$$

avec

I = intensité du courant traversant les électrodes

t = temps pendant lequel le courant d'intensité I traverse la cellule.

Parmi les électrolytes susceptibles d'être mis en œuvre, les bromures sont les plus avantageux, ainsi qu'il résulte d'un certain nombre d'expériences faites dans le cadre de l'oxydation de l'isosorbide avec d'autres sels (concentration en isosorbide de 1,46% en poids, pH de 7, densité de courant de 25 A/dm$^2$, température de 19 °C et quantité d'électricité expérimentale de 300 000 coulombs par mole) et dont les résultats sont réunis dans le tableau I.

Tableau I

| Nature du sel | Concentration % en poids | Rendement % | Rendement faradique % |
|---|---|---|---|
| NaBr | 1,03 | 57 | 37 |
| | 3,09 | 71 | 47 |
| KBr | 1,19 | 50 | 32 |
| KI | 3,32 | 20 | 13 |
| NaCl | 2,34 | 13 | 8 |
| $Na_2SO_4$ | 0,7 | 0 | 0 |

Dans le tableau II, on a réuni les valeurs enregistrées en faisant varier la concentration en NaBr, les autres conditions étant celles de la série d'expériences précédente.

Tableau II

| Concentration en NaBr % en poids | Rendement % | Rendement faradique % |
|---|---|---|
| 1,03 | 57 | 33 |
| 3,09 | 67 | 43 |
| 5,15 | 73 | 47 |
| 10,3 | 77 | 52 |

Il résulte de ce tableau qu'au-delà d'une concentration de 5% en NaBr, le gain en rendement est faible.

Le réglage du pH aux valeurs préférentielles susmentionnées est déterminant, ainsi qu'il résulte d'un ensemble d'expériences pour lesquelles les autres conditions variables sont fixées comme suit:

concentration en isosorbide: 1,46% en poids

concentration en NaBr: 3,09% en poids

température: 19 °C

densité de courant: 25 A/dm$^2$

quantité d'électricité

expérimentale: 300 000 coulombs/mole,

le pH étant amené aux différentes valeurs souhaitées par addition de NaOH.

En effet, des résultats réunis dans le tableau III ci-après

Tableau III

| pH | Rendement % | Rendement faradique % |
|---|---|---|
| 2 | 0 | 0 |
| 4 | 0 | 0 |
| 5 | 13 | 8,5 |
| 6 | 43 | 27 |
| 7 | 73 | 47 |
| 9 | 23 | 14 |
| 10 | 8 | 5 |
| 12 | 0 | 0 |

on constate qu'à partir de pH 8 à pH 9, il y a une nette baisse de rendement, due à la décomposition de la monocétone.

On constate par ailleurs que, si le pH est initialement fixé à 7, il a tendance à baisser pour tendre vers 5, valeur à laquelle le rendement a déjà sensiblement baissé.

La surveillance du pH et son maintien le plus près possible de 7 sont donc importants.

Une autre série d'expériences, destinées à mettre en évidence le rôle de la température de la solution, montre qu'effectivement la température ambiante et notamment la température de 19 °C est la plus favorable, les paramètres fixes étant:
concentration en isosorbide: 1,46% en poids
concentration en NaBr: 3,09% en poids
pH: 7
densité de courant: 25 A/dm²
quantité d'électricité
expérimentale: 300 000 coulombs/mole.
Les résultats sont réunis dans le tableau IV.

Tableau IV

| Température en °C | Rendement % | Rendement faradique % |
|---|---|---|
| 5 | 64 | 41 |
| 15 | 66 | 41 |
| 19 | 71 | 47 |
| 40 | 51 | 34 |
| 60 | 47 | 30 |

La densité de courant appliquée ne constitue pas une grandeur très critique, comme il résulte du groupe d'expériences suivant dans lequel les paramètres fixes sont:
concentration en isosorbide: 1,46% en poids
concentration en NaBr: 5,15% en poids
température: 19 °C
pH: 7
quantité d'électricité
expérimentale: 300 000 coulombs/mole,
la densité de courant variant de 2,5 à 45 A/dm².
Les résultats sont réunis dans le tableau V.

Tableau V

| Densité de courant A/dm² | Rendement % | Rendement faradique % |
|---|---|---|
| 2,5 | 71 | 47 |
| 7 | 70 | 45 |
| 15 | 67 | 43 |
| 25 | 71 | 47 |
| 45 | 73 | 48 |

Le choix d'une quantité d'électricité expérimentale de 400 000 à 500 000 coulombs par mole est retenu en raison du fait qu'en dessous de 400 000 coulombs par mole, le rendement est trop faible et qu'au-dessus de 500 000 coulombs par mole, le bénéfice de l'augmentation du rendement est contrebalancé par l'apparition de produits secondaires.

On a réalisé, à ce propos, une série d'expériences en faisant varier la quantité d'électricité et en conservant les paramètres fixes suivants:
concentration en isosorbide: 1,46% en poids
concentration en NaBr: 1,03%
température: 19 °C
pH: 7
densité de courant: 25 A/dm².
Les résultats sont réunis dans le tableau VI.

Tableau VI

| Quantité d'électricité expérimentale coulombs/mole | Rendement % | Rendement faradique % |
|---|---|---|
| 0 | 0 | 0 |
| 14 000 | 7 | 97 |
| 50 000 | 16 | 65 |
| 100 000 | 28 | 52 |
| 200 000 | 45 | 42 |
| 300 000 | 61 | 38 |
| 400 000 | 73 | 34 |
| 500 000 | 81 | 30 |

A la lecture de ce tableau, on constate que le rendement de la réaction et le rendement faradique varient en sens contraire.

D'après les mesures effectuées, on a, pour une quantité d'électricité de 450 000 coulombs/mole, un rendement de 77% et un rendement faradique de 32%, ce qui correspond à des conditions particulièrement intéressantes du point de vue économique.

Concernant enfin le dernier paramètre, à savoir la concentration de la solution en dianhydrohexitol, on a pu le choisir dans une plage relativement large dont la limite supérieure correspond à un seuil au-delà duquel la réaction, bien qu'encore possible, est perturbée par des phénomènes de diffusion.

A l'intérieur de la plage de concentrations indiquée, la variation de la concentration en dianhydrohexitol n'a pas une très grande influence, ainsi qu'il résulte de la série d'expériences effectuées en faisant varier ladite concentration ainsi que la quantité d'électricité pour certaines concentrations, les paramètres fixes étant:
concentration en NaBr: 1,03% en poids
température: 19 °C
pH: 7
densité de courant: 25 A/dm².
Les résultats enregistrés ont été consignés dans le tableau VII.

Tableau VII

| Concentration en % en poids | Quantité d'électricité expérimentale en coulombs/mole | Rendement % | Rendement faradique % |
|---|---|---|---|
| 0,73 | 600 000 | 42 | 14 |
| 1,46 | 300 000 | 61 | 38 |
| 7,3 | 150 000 | 32 | 97 |
| 15,8 | 8 300 | 4 | 97 |
| | 33 300 | 15 | 87 |
| | 66 600 | 23 | 68 |
| | 122 500 | 39 | 59 |
| | 245 800 | 59 | 46 |
| | 450 000 | 78 | 30 |
| 24,8 | 64 000 | 19 | 65 |
| | 78 000 | 25 | 62 |
| | 236 000 | 52 | 44 |

+ produits secondaires

Ceci étant, on donne ci-après quelques exemples numériques illustrant le procédé conforme à l'invention.

Exemple 1

Préparation du 1,4-3,6-dianhydro-L-sorbose.

On a recours à une cellule non divisée en compartiments anodique et cathodique et équipée de deux électrodes en platine, d'une électrode de référence au calomel saturé, d'un agitateur magnétique, d'une électrode de verre reliée à un pH-mètre et d'une enveloppe thermostatée.

On effectue deux expériences.

La température étant fixée à 19 °C, on verse dans la susdite cellule 100 $m^3$ d'une solution aqueuse contenant, dans la première expérience, 1,42 g (9,7 mmoles) d'isosorbide et, dans la deuxième expérience, 29,7 g (20,3 mmoles) d'isosorbide, la quantité de NaBr étant de 1,03 g (10 mmoles).

On applique dans les deux cas une tension de 20 V, la densité de courant étant de 25 A/$dm^2$. Le pH varie de 7 à 5. On arrête chaque fois l'expérience quand la quantité d'électricité ayant traversé la cellule est égale à 500 000 coulombs/mole.

Après élimination du bromure de sodium (séparation par chromatographie liquide) et évaporation de l'eau, on recueille dans les deux expériences correspondant, dans la première expérience, à un rendement en monocétone de 79% et un rendement faradique de 30% et, dans la deuxième expérience, à un rendement de 79,5% et un rendement faradique de 30%.

Exemple 2

Oxydation des hydroxyles endo de l'isomannide.

En utilisant l'appareillage de l'exemple 1, on traite 100 $m^3$ d'une solution aqueuse d'isomannide d'une concentration de 1,46%.

La concentration en NaBr est de 5,15%.

La densité de courant appliquée aux électrodes est de 15 A/$dm^2$, la température de la solution est de 19 °C et le pH de 7. On arrête l'expérience quand 300 000 coulombs/mole ont traversé la cellule.

Les rendements en mono- et dicétone sont respectivement de 49% (0,71 g) et de 32% (0,48 g).

## Revendications

1. Procédé de préparation des dérivés cétoniques de l'isomannide et de l'isosorbide, caractérisé par le fait que l'on soumet à une électrooxydation anodique une solution aqueuse d'isomannide ou d'isosorbide comportant un bromure alcalin à titre d'électrolyte, le pH de cette solution étant de 6 à 8.

2. Procédé selon la revendication 1, caractérisé par le fait que l'électrooxydation anodique est mise en œuvre en présence de NaBr à une concentration de 0,5 à 11% en poids sur une solution d'isomannide ou d'isosorbide d'une concentration de 0,5 à 40%, la température de cette solution étant de 10 à 60 °C et la quantité d'électricité appliquée de 50 000 à 500 000 coulombs par mole de dianhydrohexitol traité.

3. Procédé selon la revendication 2, caractérisé par le fait que le NaBr est présent à une concentration de 1 à 5% en poids.

4. Procédé selon l'une des revendications 2 et 3, caractérisé par le fait que la solution d'isomannide ou d'isosorbide est d'une concentration de 15 à 25% en poids.

5. Procédé selon l'une des revendications 2 à 4, caractérisé par le fait que la température est de 15 à 20 °C.

6. Procédé selon l'une des revendications 2 à 5, caractérisé par le fait que la quantité d'électricité appliquée est de 300 000 à 500 000 coulombs par mole de dianhydrohexitol traité.

7. Procédé selon l'une des revendications 1 à 6, caractérisé par le fait que la réaction est mise en œuvre dans une cellule électrolytique comportant des électrodes en platine ou en carbone.

8. Procédé selon la revendication 7, caractérisé par le fait que les compartiments anodique et cathodique de la cellule électrolytique sont séparés par un diaphragme.

## Patentansprüche

1. Verfahren zur Herstellung von Ketonderivaten des Isomannids und Isosorbids, dadurch gekennzeichnet, dass man eine wässrige Isomannid- oder Isosorbidlösung, die ein Alkalibromid als Elektrolyt enthält, bei einem pH dieser Lösung von 6 bis 8 einer anodischen Elektrooxidation unterzieht.

2. Verfahren gemäss Anspruch 1, dadurch gekennzeichnet, dass die anodische Elektrooxidation in Anwesenheit von NaBr in einer Konzentration von 0,5 bis 11 Gew.-% an einer Isomannid- oder Isosorbidlösung mit einer Konzentration von 0,5 bis 40% durchgeführt wird, wobei die Temperatur dieser Lösung 10 bis 60 °C beträgt, und die aufgewendete Elektrizitätsmenge 50 000 bis 500 000 Coulomb je Mol behandeltes Dianhydrohexit beträgt.

3. Verfahren gemäss Anspruch 2, dadurch gekennzeichnet, dass das NaBr in einer Konzentration von 1 bis 5 Gew.-% vorliegt.

4. Verfahren gemäss einem der Ansprüche 2 und 3, dadurch gekennzeichnet, dass die Isomannid- oder Isosorbidlösung eine Konzentration von 15 bis 25 Gew.-% besitzt.

5. Verfahren gemäss einem der Ansprüche 2 bis 4, dadurch gekennzeichnet, dass die Temperatur 15 bis 20 °C beträgt.

6. Verfahren gemäss einem der Ansprüche 2 bis 5, dadurch gekennzeichnet, dass die aufgewendete Elektrizitätsmenge 300 000 bis 500 000 Coulomb je Mol behandeltes Dianhydrohexit beträgt.

7. Verfahren gemäss einem der Ansprüche 1 bis 6, dadurch gekennzeichnet, dass die Reaktion in einer Elektrolysezelle mit Platin- oder Kohlenstoffelektroden durchgeführt wird.

8. Verfahren gemäss Anspruch 7, dadurch gekennzeichnet, dass die Anoden- und Kathodenkammern der Elektrolysezelle durch ein Diaphragma getrennt sind.

**Claims**

1. Process for the preparation of ketone derivatives of isomannide and of isosorbide, characterized by the fact that an aqueous isomannide or isosorbide solution comprising an alkaline bromide as electrolyte and having a pH of 6 to 8 is subjected to anodic electrooxidation.

2. Process according to claim 1, characterized by the fact that the anodic electrooxidation is carried out in the presence of NaBr at a concentration of 0.5 to 11 wt.% on an isomannide or isosorbide solution of a concentration of 0.5 to 40%, the temperature of the said solution being from 10 to 60 °C and the quantity of electricity applied being from 50 000 to 500 000 coulombs per mole of dianhydrohexitol treated.

3. Process according to claim 2, characterized by the fact that the NaBr is present at a concentration of 1 to 5 wt.%.

4. Process according to claim one of claim 2 and 3, characterized by the fact that the concentration of the isomannide or isosorbide solution is from 15 to 25 wt.%.

5. Process according to one of claims 2 to 4, characterized by the fact that the temperature is from 15 to 20 °C.

6. Process according to one of claims 2 to 5, characterized by the fact that the amount of electricity applied is from 300 000 to 500 000 coulombs per mole of dianhydrohexitol treated.

7. Process according to one of claims 1 to 6, characterized by the fact that the reaction is carried out in an electrolytic cell comprising platinum or carbon electrodes.

8. Process according to claim 7, characterized by the fact that the anode and the cathode compartments of the electrolytic cell are separated by a diaphragm.